# EUROPEAN PATENT SPECIFICATION

(11) **EP 2 856 986 B1**
(45) Date of publication and mention of the grant of the patent: **14.10.2020**
(21) Application number: 14186964.4
(22) Date of filing: 30.09.2014
(51) Int. Cl.: A61F 7/00, A61F 7/02

(54) **Cosmetic method for reducing subcutaneous adipose tissue**
Kosmetisches Verfahren zur Reduzierung von subkutanem Fettgewebe
Procédé cosmétique pour réduire le tissu adipeux sous-cutané

(30) Priority: 03.10.2013 ES 201331451
(43) Date of publication of application: 08.04.2015
(73) Proprietor: Clinipro, S. L., 08173 Sant Cugat del Valles (ES)
(72) Inventor: Plan, Philippe, 08173 Sant Cugat del Vallès (ES); Pinto, Hernán, 08173 Sant Cugat del Vallès (ES)
(74) Representative: Gallego Jiménez, José Fernando

(56) References cited:
- WO-A1-2013/026393
- US-A1- 2009 069 795
- US-A1- 2010 036 295

## Description

### Technical field

The present invention relates to the field of cosmetic methods for reducing subcutaneous adipose tissue.

### Technical Background

Adipose tissue or fat tissue is the tissue formed by the association of cells that store fat inside them: the adipocytes. In the human body there are two types: the white adipose tissue and the brown adipose tissue. This adipose tissue is located in specific regions, which are known as adipocyte deposits, and is located under the skin (subcutaneous fat), around the internal organs (visceral fat), in the bone marrow (yellow bone marrow) and in the breasts. This tissue has both mechanical functions (for example, the protection of internal organs) and metabolic functions (for example, responsible for generating energy for the organism).

Adipose tissue is one of the most abundant tissues and accounts for about 15-20% of the body weight in men and 20-25% of the body weight in women.

Since ancient times, human beings have been developing methods that would allow reducing the accumulation of adipose tissue in unwanted parts of their own body for aesthetic reasons. Treatments can be systemic, such as the use of gastric balloons, or localized, which are directed to the reduction of localized body fat. Such localized procedures can be divided between those using invasive techniques, and those using noninvasive techniques.

Liposuction is one of the most used invasive techniques. Liposuction is a surgical procedure that consists in the removal of extra fat or adipose tissue in the affected area of the body, by its aspiration with thin cannulas that are inserted through small incisions in the fatty deposits located under the skin. Such cannulas are connected to a suction machine for mechanical liposuction, or by ultrasound, for ultrasonic liposuction. With the suction of the extra fat, a shaping of the treated area is achieved with a consequent improvement in the figure and outline. However, since it is a surgical procedure, this technique is not without risk, since complications may arise, for example a hemorrhage.

Within the noninvasive techniques are, for example, the treatments with ultrasounds, radiofrequencies or microwaves, and thermal treatments at different temperatures.

Thus, in the web page http://www.biotecitalia.com/en/fusiomedice-eng.html, a system for treating local fat and cellulite is described, in which five different technologies are used: monopolar and bipolar radiofrequency, low frequency cavitational ultrasound, sub-dermal vacuum massage, and liposhock. In the latter procedure, the area to be treated is suctioned, and the tissue is exposed to a sequence of high temperature (60° C) and low temperature (-10° C) that triggers an apoptosis process in the adipocytes, leading to their physiological natural death.

In the U.S. patent application US-A-2009/0069795, a method is described for destroying a particular tissue, for example adipose tissue, by heating, without the surrounding tissue being affected. The method includes:
a) heating the target tissue and the surrounding tissue at a temperature lower than the one required for destroying them, wherein the target tissue has a longer thermal relaxation time than the surrounding tissue,
b) letting the tissues cool for a period of time, wherein the target tissue does not cool down as much as the tissue that surrounds it, and
c) reheating the tissues, wherein the target tissue is destroyed, and the surrounding tissue is not.

In the U.S. patent application US-A-2009/0157152, a cosmetic method to improve the state of the skin in the face and/or in the neck is described. This method comprises a step of thermal stimulation at a temperature preferably comprised between 41° C and 43° C for a period of time preferably comprised between 60 and 90 minutes.

In the U.S. Patent US5507790, a noninvasive method for the reduction of subcutaneous adipose tissue deposits in humans is described by using an accelerated lipolysis metabolism. Said method includes the use of heat alone or in combination with a suitable lipolytic drug. It is also described that the adipose tissue is heated at a temperature comprised between 40° C and 41.5º C, but not above 42° C, and this temperature is maintained for a period of up to 20-25 minutes.

In the international patent application WO-A-01/32091, a method for noninvasive removal of adipose tissue in humans is described. In this method heat is applied to raise the temperature of the adipocytes between 41° C and 46° C for a period comprised between 5 minutes and 2 hours. The thermal energy can be administrated by ultrasounds, radiofrequency or microwaves.

In the international patent application WO-A-02/087700, a method is described for the treatment of a selected area of the skin and/or the subcutaneous tissue comprising heating said area at a temperature comprised between 32° C and 50° C for a certain period of time comprised between 20 seconds and 24 hours, preferably between 30 minutes and 8 hours, and administering a composition comprising an active ingredient, for example, a lipolytic compound such as caffeine.

In the international patent application WO-A-03/078596, it is described the selective disruption of adipose cells by the application of a cooling gradient at the skin surface without causing damage to the other cells.

In the article Avram et al., Cryolipolysis™ for subcutaneous fat layer reduction, Lasers Surg. Med., 2009, 41, 703-708, it is disclosed that cryolipolysis is a noninvasive method for the selective reduction of adipocytes by localized and controlled cooling thereof. It is also indicated that phagocytosis of the lipids lasts 90 days or more, and that after this period a clear reduction of the thickness of the fat layer is observed.

In the international patent application WO-A 2012/075419, it is described a method for the disruption of subcutaneous adipose tissue by using heat, but it is accompanied by cooling of the area to prevent damages to the near epithelial or vascular tissues. It is also described that the cooling can occur simultaneously to the heat treatment, and that can be extended after finishing it in order to reduce inflammation and pain. Hyperthermia treatment of the adipose tissue raises its temperature above 40º C, preferably between 42° C and 46° C, while the temperature of the surrounding tissues is maintained below 40° C.

In the international patent application WO-A-2013/026393, a method for losing weight by reducing fat is described comprising locally heating an area that requires the reduction of fat at a temperature comprised between 37° C and 60° C until the blood and muscles are separated from the fat, and then locally freeze this area at a temperature comprised between -10°C and 5°C to solidify the fat.

Documents US2010036295 A1 and US2008077211 A1 disclose further relevant background art.

None of the proposed solutions satisfactorily solves the problem of the effective removal of subcutaneous fat deposits localized in the human body.

Thus, there remains a need for a noninvasive cosmetic method, to effectively reduce the fat localized in such deposits and thus improve the aesthetic appearance of people.

### Summary of the invention

The object of the present invention is a cosmetic method according to the appended independent claim 1 for reducing the subcutaneous adipose tissue. Preferred embodiments are disclosed in the dependent claims.

### Description of the figures

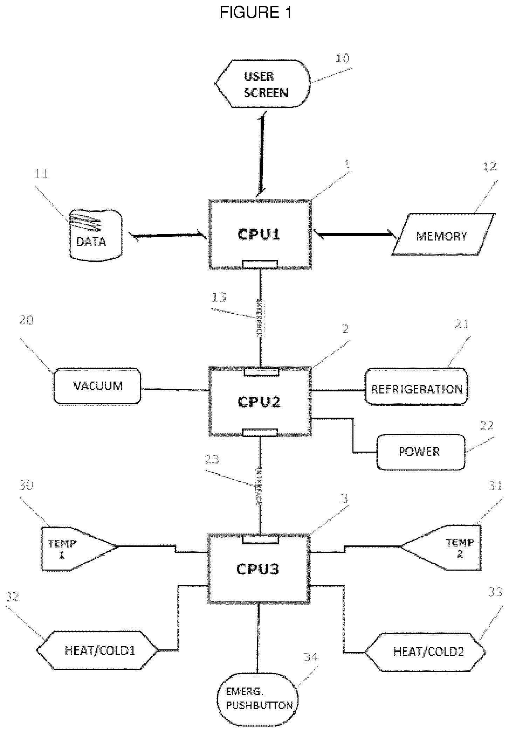

Figure 1 In Figure 1 a diagram of the internal units and the associated functions thereof is represented, which includes a particular embodiment of the device 1 not forming part of the invention, for performing the method of the invention. Elements 1, 2 and 3 are three CPUs processing units that control the functioning of the equipment. Elements 10, 11 and 12 are, respectively, a touchscreen to establish communication with the device, an internal memory necessary for the execution of all the processes and an internal database that generates a register of incidents to facilitate the diagnosis of a possible breakdown. Elements 13 and 23 are interfaces that communicate the different CPUs. Elements 20, 21 and 22 are, respectively, a vacuum system with feedback sensor, to obtain a better result in the treatment by introducing in the cavity of the device the maximum adipose tissue possible, a liquid cooling system to compensate the temperature generated in the heat/cold elements, and a switch for the power flow necessary for CPU3 (3). Elements 30 and 31 are temperature sensors to regulate the operating conditions of the internal elements of the equipment. Units 32 and 33 are the heat and cool generating units according to the step of the method. Element 34 is an emergency shutdown pushbutton.

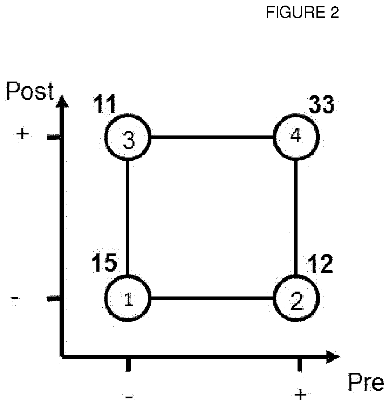

Figure 2 Figure 2 shows the 2² factorial design with the factors: Pre-heating on the ordinates and post-heating on the abscissa, wherein the sign "-" means that the treatment is not performed and the "+" sign means that the treatment is performed. Each of the numbers that are located within a circle correspond to the test described in Example 1, and the numbers that are located outside of the circle correspond to the adipocytes mortality, expressed as a percentage of the total adipocytes. A synergistic effect is observed by combining a pre-heating step with a cooling step and a post-heating step.

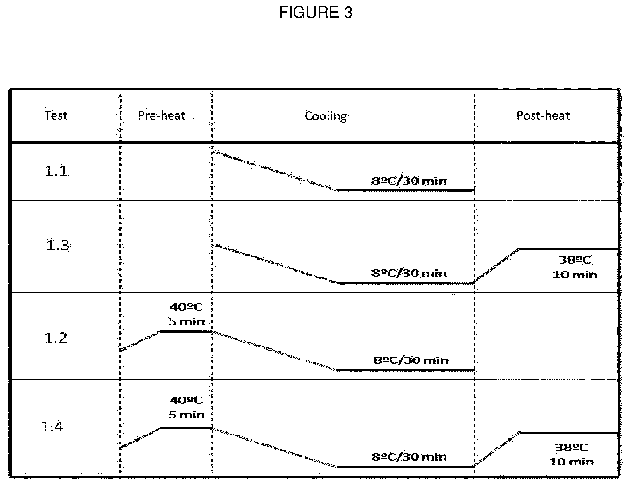

Figure 3 In Figure 3 it is schematically represented the time and temperature profiles for the treatment of the method of the invention (test 1.4) described in Example 1 as well as for the comparative treatments (tests 1.1 - 1.3) described therein. The ordinate represents the temperature, and the abscissa the time.

### Detailed description of the invention

The invention has as object a cosmetic method for reducing the subcutaneous adipose tissue in a localized area of an adult human body , the method consists of the following steps:
a) heating a localized area of the human body with heating means that are at a temperature of not more than 45° C,
b) subsequently, cooling this area with cooling means that are at a temperature comprised between 2°C and 6°C, and
c) subsequently, heating this area with heating means that are at a temperature of not more than 45° C.

The authors of the present invention have developed a noninvasive cosmetic method for reducing the subcutaneous adipose tissue in localized areas of an adult human body that has a high efficacy in the immediate reduction of the number of adipocytes, as well as a high effect of this reduction deferred in time, and it maintains a satisfactory metabolic functionality of the remaining adipocytes.

As already stated, the temperature indicated in the steps of the method of the invention corresponds to the temperature of the heating/cooling means that are in contact with the skin, not to the temperature of the adipocytes. These means allow heating or cooling, depending on the step of the treatment, the adipocytes at a temperature that is closely related to the temperature thereof. For example, when the temperature of the heating/cooling means is 3.1º C, the adipocytes are below 10.4°C, after a period of 25-35 minutes, as described in Pinto et al., A study to evaluate the action of lipocryolysis, CryoLetters, 2012, 33 (3), 176-180, and when the means are at 42° C, the adipocytes are approximately at 38° C after a period of time comprised between 5-10 minutes.

### The cosmetic method

The method of the invention is a noninvasive cosmetic method. This is a non-traumatic method that acts from the surface of the skin and does not invade the body through incisions to reduce subcutaneous adipose tissue. In this method it is used a temperature range that can be considered substantially safe for human body, since temperatures below 2° C or above 45° C are not used. The method is applied topically to the area having an excessive amount of subcutaneous adipose tissue and for this is not necessary any knowledge of medicine, but it can be carried out by people not requiring a degree in medicine. This method does not include any surgical step, and neither any therapeutic effect is produced. The result of applying the method of the invention leads to an aesthetic effect by reducing the subcutaneous adipose tissue located in specific areas of the body. The effects of this method are purely cosmetic because they can only be observed in the superficial layers of adipose tissue. Furthermore, as discussed below, the method of the invention maintains the functionality of the adipocytes which have not been affected by it.

The method of the invention differs substantially from invasive methods for reducing fat, such as, for example, liposuction, that can have side effects on the human health, since it involves a strong interaction with the human body.

The method of the invention can be applied easily; it is applied directly to the skin through a device that, optionally, makes a vacuum effect in order to better get a skin fold with localized subcutaneous fat tissue and, in turn, it allows the heating and cooling of this area at temperatures previously stated.

This method includes some temperature profiles with which the lipid-rich cells are altered, and simultaneously the non-lipid-rich cells are not altered.

The method of the invention can be applied more than one session. In fact, it is usually applied on more than one occasion, being usually the period between sessions of approximately 45 days.

The method of the invention produces an almost imperceptible effect after its application to the localized area of the human body. The effects of this treatment begin to appear after a period generally comprised between 15 and 30 days. This is because the adipocytes that have been object of the method initiate a process of apoptosis, being enveloped and digested by macrophages and eliminated by natural means during the days following the treatment. The repeated application of the method allows to significantly reduce the subcutaneous fat deposits.

### Localized area of the human body

The method of the invention is used to reduce the subcutaneous adipose tissue of a localized area of an adult human body, preferably in the abdomen, waist, back, arms and thighs.

In the context of the invention an adult human body is considered to be the one corresponding to a person with an age of at least 16 years approximately.

The method of the invention is not suitable for reducing the adipose tissue that is located in a generalized manner in the human body.

Preferably the localized area of the human body is introduced into an appropriate elongated cavity for housing the fold of skin on which the method of the invention is performed. In a more preferred embodiment, this cavity includes an absorption hole for vacuum application in order to achieve a greater efficacy in the reduction of the adipose tissue, since this way the blood flow to this area is restricted.

### Pre-heating step

The method of the invention comprises an initial heating step (pre-heating) of the localized area of the human body that shows an excess of subcutaneous adipose tissue.

The heating of this area can be performed by conventional heating means such as those described, for example, in the U.S. patent US5507790. In the method of the invention the heating of the localized area is carried out by using radiofrequency, microwaves, ultrasounds or a thermoelectric device based on the Peltier effect; preferably is used a thermoelectric device based on the Peltier effect.

The Peltier effect is a thermoelectric property that is manifested when electric current flows through a circuit formed by two types of conductive substances. In the circuit, one of the parts is colder than the other, and the difference of temperature generated depends on the intensity of the current flowing through the circuit.

The heating of the localized area is performed until reaching a temperature of not more than 45° C in the heating means that are in contact with the skin. Preferably, the temperature is not more than 44º C, more preferably not more than 43°C, and still more preferably not more than 42°C.

This temperature of the heating means is maintained generally a period of time comprised between 2 minutes and 10 minutes, preferably between 3 minutes and 8 minutes, more preferably between 4 and 6 minutes, and more preferably 5 minutes.

Typically the heating means in the pre-heating step are at room temperature and the heating rate from this temperature to the desired temperature is comprised between 1°C/min and 8° C/min, preferably between 1°C/min and 6º C/min, and more preferably between 1°C/min and 3° C/min. The estimated time to bring the heating means to the stated temperature is comprised between 1 and 20 minutes.

### Cooling step

The process of the invention comprises a step of cooling of the localized area of the human body that has an excess of subcutaneous adipose tissue. This cooling step is performed after the pre-heating step.

The cooling of this area can be carried out by conventional cooling means as those described, for example, in the international patent application WO-A-2012/075419, in which the use of a cold air stream, or the contact with a cold solution are mentioned, or in the international patent application WO-A-00/44346, where some compositions appropriate to reduce the temperature of the skin of a person are described. In the method of the invention the cooling means preferably use cold solutions, cold air, or a thermoelectric device based on the Peltier effect; more preferably is used a thermoelectric device based on the Peltier effect.

The cooling of the localized area is performed until reaching a temperature comprised between 2°C and 6°C in the cooling means in contact with the skin. Preferably the temperature is comprised between 3° C and 5° C, and more preferably between 3° C and 4° C.

Once the cooling means reach the stated temperature, it is maintained for a minimum period of time of 5 minutes, preferably between 5 minutes and 60 minutes, more preferably between 8 minutes and 50 minutes, more preferably between 10 minutes and 40 minutes, and still more preferably between 20 minutes and 30 minutes.

Usually the rate of cooling from the initial temperature, at which the cooling means are, to the desired temperature is comprised between 1° C/min and 8° C/min, preferably between 1°C/min and 6º C/min, and more preferably between 1°C and 3° C. The estimated time to bring the cooling means to the stated temperature is comprised between 2 and 30 minutes.

### Post-heating step

The method of the invention comprises a heating step (post-heating) of the localized area or the human body that has an excess of subcutaneous adipose tissue after the cooling step.

As already mentioned above, in the method of the invention the heating of the localized area is carried out by using radiofrequency, microwaves, ultrasounds or a thermoelectric device based on the Peltier effect; preferably is used a thermoelectric device based on the Peltier effect.

The heating of the localized area is performed until reaching a temperature of not more than 45° C. This temperature refers to the temperature in the heating means that are in contact with the skin. Preferably the temperature is not more than 44º C, more preferably not more than 43° C, and still more preferably not more than 42°C.

Once the heating means reach the stated temperature, it is generally maintained a period of time comprised between 2 minutes and 20 minutes, preferably between 5 minutes and 15 minutes, more preferably between 8 and 12 minutes, and still more preferably 10 minutes.

Usually the heating rate from the initial temperature, at which the heating means are, to the desired temperature is comprised between 1°C/min and 8° C/min, preferably between 1°C/min and 6º C/min, and more preferably between 1°C/min and 3° C/min. The estimated time to bring the heating means to the stated temperature is comprised between 1 and 20 minutes.

In order to control temperatures in the skin, the method of the invention can include the use of a feedback device in the form of a sensor, as described in the patent application GB-A-2286660.

A particularly preferred embodiment of the method of the invention consists of the following steps:
a) heating a localized area of the human body with heating means that are at a temperature of not more than 44º C, for a period comprised between 2 and 10 minutes,
b) subsequently, cooling this area with cooling means that are at a temperature comprised between 3° C and 5° C for a period comprised between 5 and 60 minutes, and
c) subsequently, heating this area with heating means that are at a temperature of not more than 44º C for a period comprised between 2 and 20 minutes.

A particularly preferred embodiment of the method of the invention consists of the following steps:
a) heating a localized area of the human body with heating means that are at a temperature of not more than 42º C, for a period comprised between 4 and 6 minutes,
b) subsequently, cooling this area with cooling means that are at a temperature comprised between 3° C and 4° C for a period comprised between 10 and 40 minutes, and
c) subsequently, heating this area with heating means that are at a temperature of not more than 42º C for a period comprised between 8 and 12 minutes.

Surprisingly it has been observed that with the method of the invention it is possible to obtain an immediate reduction in the number of adipocytes by direct apoptosis, a deferred reduction thereof by crystal formation, and maintain the functional viability of the adipocytes so that they maintain a substantially normal level of lipolysis. The combination of the steps of pre-heating, cooling and post-heating allows to obtain a significant increase in the direct apoptosis of adipocytes in comparison with the methods that only include one or two of such steps, whilst the crystal formation remains comparable to that obtained by employing said alternative procedures.

### Device (not forming part of the invention)

The device suitable for carrying out the method of the invention can be, for example, the one described in the patent application GB-A-2286660. Thus, this device comprises one or more thermoelectric units based on the Peltier effect, a heat exchanger to dissipate the cold generated in the heating steps and the heat generated during the cooling step, a container of liquid to cool or heat the heat exchanger, a pump for recirculating this liquid between the container and the heat exchanger, and a cooling/heating unit to cool this liquid.

In a preferred embodiment the heat and cool generation is carried out by using one or more thermoelectric units based on the Peltier effect.

The device can further incorporate a screen as a user interface, to report in which state is the device when the method of the invention is performed. Preferably, it is a touch screen and therefore, the process parameters can be configured, it is also possible to execute the stop and the start of the treatments, the monitoring of each of the variables, and information about the status and situation of each cycle of the method.

The thermoelectric units are preferably located in a head comprising an elongated cavity suitable for housing the fold of skin on which the method of the invention is performed. In a more preferred embodiment this cavity includes an absorption hole for vacuum application in order to achieve greater efficacy in the reduction of the adipose tissue. This head may include several light indicators of the LED type to inform about the function which is being performed during the procedure.

The device can advantageously include process control units for controlling the different elements involved in the method of the invention. In Figure 1 it is shown a possible embodiment of a diagram that includes the internal units of the device and the functions associated therewith. According to this embodiment, the beginning of the procedure is performed by the user pressing the "start" button placed in the user screen (10), selecting next the thickness of the adipose tissue, and then eventually the command to start the vacuum is given, from the screen (10). From this moment the CPU corresponding to the unit (2) receives the command from the interface and activates the suction (20) with a pre-assigned regulation level according to the adipose tissue. The implemented software can work on any standard operating system as, for example, WindowsCE or GNU/Linux, and automatically manages all the internal processes in the memory (12) and stores the necessary information to use the equipment, also generating internal error codes in the database (11) to facilitate repair operations in case of the eventual breakdowns.

The system (2) detects when the internal cavity of the applicator has the correct vacuum (20) enabling in the screen (10) the beginning of the therapy, at that moment a start session command is sent to the cooling/heating system (32)/(33), refrigeration system (21) and vacuum (20). The type of therapy and the sequences of heat/cool are determined by the CPU1 (1) sending the necessary commands by internal communication (13) (23) to the rest of the units according to the cycle, at the same time all the internal variables for monitoring and tracking the equipment status and the effectiveness of the therapy are received. The CPU2 (2) monitors the power delivered at the CPU3 (3) during the application of heat/cold (32)/(33), in case of obtaining a deviation outside the established margins, as a possible failure of CPU3 (3), the power module (22) is disconnected to stop supplying power to the heat/cold elements (32) (33).

The CPU3 (3) is responsible for not exceeding the maximum and minimum temperature values stated, by using temperature sensors (30)/(31) in the critical areas, these being necessary to not exceed the maximum values of cooling detrimental to the internal components of the equipment or electronic components, such as the ice generation and moisture. During the treatment the necessary power for the generation of heat or cold (32)/(33) is applied depending on the point of cycle according to the commands received from the upper CPUs (1)/(2). The CPU3 also controls the emergency stop pushbutton (34) to release the order of immediately stop the session to all upper control units (1)/(2) stopping the heat/cool system, refrigeration system and vacuum, and simultaneously a stop activation message is displayed on the screen for validation by the user.

Once finished the session, the heat/cold system (32)/(33) and vacuum (20) are stopped. The device returns to the idle state and displays on the screen the start button waiting to be pressed by the user from the screen (10).

### Tests on adipocytes

Tests on isolated rat adipocytes were performed applying different thermal profiles:
1. Cooling,
2. Pre-heating combined with cooling,
3. Cooling combined with post-heating, and
4. Pre-heating combined with cooling and with post-heating.

In the tests with adipocytes, the temperatures correspond to the temperature at which the adipocytes were maintained at different steps of the procedure.

After the tests, it was observed that the adipocytes treated according to the method of the invention (profile 4) showed a significantly higher mortality rate than the mortality rate observed in the other comparative methods. This cell death is an immediate effect that occurs during the application of the method of the invention itself.

A considerable formation of beta crystals in the adipocytes is also observed, which is related to the slow death by apoptosis of the adipocytes, and that results in a deferred effect of reduction of the number of adipocytes.

In Figure 2 the design space of the tests performed is shown, and it corresponds to an experimental matrix of a factorial design 2², in which a synergistic effect is observed by combining the pre-treatment with heat, the cooling step, and the post-treatment with heat in terms of mortality of the adipocytes. It was observed that a single step of pre- or post- heating combined with cooling practically does not modify, rather reduce, the effect of the cold treatment alone.

Tests were also carried out to determine the functional viability of adipocytes from the subcutaneous adipose tissue after being subjected to the method of the invention. As described in the Examples section, these adipocytes maintained normal lipolytic activity. I.e., the adipocytes maintained a lipolytic activity and generated glycerin as a product from the hydrolysis of the fats. Lipolysis is the physiological mechanism by which adipose tissue mobilizes the energy stored as fat to make it available to cell metabolism. The fatty acids released in this process are used in many body tissues as energy substrate, alternative to glucose.

It is thus observed that, surprisingly, the cosmetic noninvasive method of the invention produces a synergistic effect by combining its component steps, and allows obtaining a superior effect in the immediate removal of the adipocytes, while maintaining a significant effect on the deferred elimination thereof, and furthermore the unaffected adipocytes continue to be functionally viable.

The examples which follow are given in order to provide the skilled in the art with a sufficiently clear and complete explanation of the present invention, but they should not be considered as limiting the essential aspects of the object of the invention, as have been exposed in the previous sections of this description.

### Examples

### Preparative example 1.- Isolation of the adipocytes

To conduct the method of the invention it was proceeded to obtain murine white adipose tissue.

Seven Sprague Dawley male rats of 56 days of age were anesthetized, euthanized by cervical dislocation, and white adipose tissue from the retroperitoneal area of the animals was obtained.

Approximately 2 g of white adipose tissue were digested with 10 mg of collagenase (Collagenase Type4, Worthington) in 20 ml of Krebs buffer (Hepes 1.25 mM, NaCl 12 mM, KCI 0.6 mM, MgSO₄·7H₂O 0.12 mM, CaCl₂ 0.1 mM, 2 g albumin and 0.045 g of glucose). The tissue was incubated at 37 °C in a bath with mild stirring for 40 minutes. The digestion was stopped by addition of 2 ml of EDTA 1 mM, and incubation was continued for 5 additional minutes. The sample was filtered and the isolated adipocytes were collected in a syringe (without piston), which was stoppled with a stopcock. The syringe was kept in a vertical position for 5 minutes to facilitate the flotation of the adipocytes in the buffer. The infranatant buffer was removed and the isolated adipocytes were washed twice with 10 ml of Krebs buffer. Finally, the adipocytes were resuspended in 2.5 mL of Krebs buffer.

### Example 1.- Effects of the exposure of adipocytes to different thermal treatments

For each thermal treatment, the adipocytes isolated in Preparative Example 1 were distributed in 12 vials: 6 vials for the adipocytes belonging to the control group, and 6 vials for the treated group. In each group, 3 vials were used for samples without isoproterenol (ISO-) and 3 vials for samples with isoproterenol (ISO+). This medicament was used to stimulate the lipolysis in the adipocytes and determining their functional viability.

Each vial contained 150 µL of the suspension of adipocytes. To conduct the counting of adipocytes, 25 µL of each of the vials were diluted 1/40 or 1/60 in Krebs buffer. After applying the corresponding experimental conditions in each case, the cell count was performed in the same way.

Simultaneously, the cells that had crystals were counted in all the samples by using a bright field microscope (Olympus CH-2) at 40x and 100x magnifications with a polarizing filter.

The thermal treatments to which the vials with adipocytes were subjected are described in Table I:

**TABLE I**

| **Test** | **Pre-heating** | | | **Cooling** | | | **Post-heating** | | |
|---|---|---|---|---|---|---|---|---|---|
| | Rate (ºC/min) | Final T (ºC) | t (min) | Rate (ºC/min) | Final T (ºC) | t (min) | Rate (ºC/min) | Final T (ºC) | t (min) |
| **1.1** | - | - | - | 2 | 8 | 30 | - | - | - |
| **1.2** | 6 | 40 | 5 | 2 | 8 | 30 | - | - | - |
| **1.3** | - | - | - | 2 | 8 | 30 | 6 | 38 | 10 |
| **1.4** | 6 | 40 | 5 | 2 | 8 | 30 | 6 | 38 | 10 |

wherein final T corresponds to the final temperature of the heating or cooling steps at which the adipocytes are maintained, and t is the time during which the adipocytes are maintained at the final T temperature.

The description of the treatments is the following:
- 1.1 (Comparative example): cooling at a rate of 2° C/minute until reaching the temperature of 8°C, which was maintained for 30 minutes.
- 1.2 (Comparative example):
   a. heating at a rate of 6° C/minute until reaching a temperature of 40° C, which was maintained for 5 minutes, and
   b. subsequently cooling at a rate of 2° C/minute until reaching the temperature of 8°C, which was maintained for 30 minutes.
- 1.3 (Comparative example):
   a. cooling at a rate of 2° C/minute until reaching the temperature of 8° C, which was maintained for 30 minutes, and
   b. subsequently heating at a rate of 6° C/minute until reaching a temperature of 38° C, which was maintained for 5 minutes.
- 1.4 (Example of the invention):
   a. heating at a rate of 6° C/minute until reaching a temperature of 40° C, which was maintained for 10 minutes,
   b. subsequently cooling at a rate of 2° C/minute until reaching the temperature of 8°C, which was maintained for 30 minutes, and
   c. subsequently heating at a rate of 6° C/min until reaching a temperature of 38°C, which was maintained for 10 minutes.

Control samples for each of the treatments were maintained at 37° C during the period of time of each of the treatments.

In Figure 3, the temperature and time profiles used in the tests described in this example are shown.

The percentage of cells that died as a result of the time elapsed during the execution of the tests is called basal mortality, and was determined from the cell count before and after the treatment in control vials.

In Table II the basal mortality detected in the control samples for each of the experimental conditions is shown:

**TABLE II**

| **Test** | **Basal mortality (%)** |
|---|---|
| 1.1 (comparative) | 4 |
| 1.2 (comparative) | -1 |
| 1.3 (comparative) | 6 |
| 1.4 | 5 |

The basal mortality was subtracted from the mortality obtained in the samples subjected to the different thermal treatments to determine the net mortality.

In the control samples the formation of beta crystals was not observed in the adipocytes corresponding to the lipid crystallization. On the contrary, in the treated samples predominantly beta type crystals were observed, but type alpha and alpha' crystals were also identified.

These four experiments were part of a factorial design 2², as shown in Table III, along with the results obtained regarding mortality of adipocytes (expressed as a percentage of dead adipocytes over the total adipocytes) and the crystal formation (expressed as a percentage of adipocytes including crystals over the total of adipocytes):

**TABLE III**

| **Test** | **Mortality (%)** | **Crystals formation (%)** |
|---|---|---|
| 1.1 (comparative) | 15 | 12 |
| 1.2 (comparative) | 12 | 25 |
| 1.3 (comparative) | 11 | 5 |
| 1.4 | **33** | **22** |

It can be observed that the adipocytes treated according to the method of the invention present a mortality rate significantly higher than the mortality rate observed in the other comparative treatments that do not include the heating-cooling-heating cycle. This cell death occurs during the application of the method of the invention itself and is an immediate effect produced by such procedure.

It is also observed a considerable formation of beta crystals in the adipocytes, which is related to their slow death by apoptosis, and that results in a deferred effect of the reduction of the number of adipocytes.

### Example 2.- Stimulation of lipolysis in the adipocytes

The samples that contained the remaining 100 µL of the suspension of adipocytes after the recounts, were diluted in a proportion 1:4 with Krebs buffer. Subsequently, 600 µL of Krebs buffer complemented with 0.2 mg/ml of adenosine deaminase (ADA, Roche) were added to the ISO- samples, and 590 µL of Krebs buffer-ADA plus 10 µL of isoproterenol (Sigma) 1 mM were added to the samples ISO+ to activate lipolysis. All the samples were incubated for 30 minutes in a thermostatic bath at 37° C. Immediately after this time, the samples were placed in an ice bath to stop the reaction. The adipocytes were separated by flotation and the infranatant was frozen at -20°C until the moment of analysis.

Given that glycerin is one of the products of lipolysis, this compound is a good indicator of this metabolic process. Thus, the content of glycerin in the obtained infranatants was determined by the *Free Glycerol Reagent* kit (Sigma). The assay is based on the transformation of the glycerol present in the medium in glycerol-1-phosphate by the action of the glycerin kinase. Then, glycerol-1-phosphate generates hydrogen peroxide by the action of the glycerolphosphate oxidase, which in turn is oxidized to a chromogen compound that produces a colored compound with an absorbance at 540 nm.

The results proved that the glycerin content in the samples stimulated with isoproterenol was significantly higher than the content in those that were not stimulated. To determine whether the stimulation of lipolysis in the samples subjected to a thermal treatment was different from the control samples kept at 37° C, the ratio between their respective net increases was calculated. The net increase is the difference between the production of glycerin in ISO+ samples and the mean value of the production of glycerin in the ISO- samples, for each experimental condition and expressed as nmoles of glycerin/10⁶ cells.

In Table IV the net percentage of stimulation of lipolysis is presented for the four treatments carried out in Example 1:

**TABLE IV**

| **Test** | **Stimulation of lipolysis (%)** |
|---|---|
| 1.1 (comparative) | 100±3 |
| 1.2 (comparative) | 74±8 |
| 1.3 (comparative) | 95±10 |
| 1.4 | 82±6 |

It can be seen that the method of the invention maintains a high functional viability of adipocytes after being subjected to the heating-cooling-heating treatment.

## Claims

1. Cosmetic method for reducing the subcutaneous adipose tissue in a localized area of an adult human body, the method consists of the following steps:
a) heating a localized area of the human body with heating means that are at a temperature of not more than 45° C,
b) subsequently, cooling this area with cooling means that are at a temperature comprised between 2°C and 6°C, **characterized by**,
c) subsequently, heating this area with heating means that are at a temperature of not more than 45° C.

2. Method according to claim 1, **characterized in that** the localized area of an adult human body is selected from the group consisting of the abdomen, waist, back, arms and thighs.

3. Method according to claims 1 or 2, **characterized in that** the heating means use radiofrequency, microwaves, ultrasounds or a thermoelectric device based on the Peltier effect.

4. Method according to claim 3, **characterized in that** the heating means use a thermoelectric device based on the Peltier effect.

5. Method according to any of claims 1 to 4, **characterized in that** the heating means are at a temperature of not more than 42° C.

6. Method according to any of claims 1 to 5, **characterized in that** the temperature in the heating means in step a) is maintained a period of time comprised between 2 minutes and 10 minutes.

7. Method according to any of claims 1 to 6, **characterized in that** the cooling means use cold solutions, cold air, or a thermoelectric device based on the Peltier effect.

8. Method according to claim 7, **characterized in that** the cooling means use a thermoelectric device based on the Peltier effect.

9. Method according to any of claims 1 to 8, **characterized in that** the cooling means are at a temperature comprised between 3º C and 4º C.

10. Method according to any of claims 1 to 9, **characterized in that** the temperature in the cooling means is maintained a period of time comprised between 5 minutes and 60 minutes.

11. Method according to any of claims 1 to 10, **characterized in that** the temperature in the heating means in step c) is maintained a period of time comprised between 2 minutes and 20 minutes.

12. Method according to claim 1, **characterized in that** it consists of the following steps:
a) heating a localized area of the human body with heating means that are at a temperature of not more than 44º C, for a period comprised between 2 and 10 minutes,
b) subsequently, cooling this area with cooling means that are at a temperature comprised between 3° C and 5° C for a period comprised between 5 and 60 minutes, and
c) subsequently, heating this area with heating means that are at a temperature of not more than 44º C for a period comprised between 2 and 20 minutes.

13. Method according to claim 1, **characterized in that** it consists of the following steps:
a) heating a localized area of the human body with heating means that are at a temperature of not more than 42º C, for a period comprised between 4 and 6 minutes,
b) subsequently, cooling this area with cooling means that are at a temperature comprised between 3° C and 4° C for a period comprised between 10 and 40 minutes, and
c) subsequently, heating this area with heating means that are at a temperature of not more than 42º C for a period comprised between 8 and 12 minutes.

## Patentansprüche

1. Kosmetisches Verfahren zur Reduzierung des subkutanen Fettgewebes in einem lokalisierten Bereich eines erwachsenen menschlichen Körpers, wobei das Verfahren aus den folgenden Schritten besteht:
a) Erwärmen eines lokalisierten Bereichs des menschlichen Körpers mit Erwärmungsmitteln, die bei einer Temperatur von nicht mehr als 45 °C liegen,
b) anschließend Abkühlen dieses Bereichs mit Kühlmitteln, die bei einer Temperatur zwischen 2 °C und 6 °C liegen, **gekennzeichnet durch**,
c) anschließend Erwärmen dieses Bereichs mit Erwärmungsmitteln, die bei einer Temperatur von nicht mehr als 45 °C liegen.

2. Verfahren nach Anspruch 1, **dadurch gekennzeichnet, dass** der lokalisierte Bereich eines erwachsenen menschlichen Körpers ausgewählt ist aus der Gruppe bestehend aus Bauch, Taille, Rücken, Armen und Oberschenkeln.

3. Verfahren nach Anspruch 1 oder 2, **dadurch gekennzeichnet, dass** die Erwärmungsmittel Hochfrequenz, Mikrowellen, Ultraschall oder eine thermoelektrische Vorrichtung, die auf dem Peltier-Effekt basiert, verwenden.

4. Verfahren nach Anspruch 3, **dadurch gekennzeichnet, dass** die Erwärmungsmittel eine thermoelektrische Vorrichtung verwenden, die auf dem Peltier-Effekt basiert.

5. Verfahren nach einem der Ansprüche 1 bis 4, **dadurch gekennzeichnet, dass** die Erwärmungsmittel bei einer Temperatur von nicht mehr als 42 °C liegen.

6. Verfahren nach einem der Ansprüche 1 bis 5, **dadurch gekennzeichnet, dass** die Temperatur in den Erwärmungsmitteln in Schritt a) über einen Zeitraum zwischen 2 Minuten und 10 Minuten aufrechterhalten wird.

7. Verfahren nach einem der Ansprüche 1 bis 6, **dadurch gekennzeichnet, dass** die Kühlmittel kalte Lösungen, kalte Luft oder eine thermoelektrische Vorrichtung, die auf dem Peltier-Effekt basiert, verwenden.

8. Verfahren nach Anspruch 7, **dadurch gekennzeichnet, dass** die Kühlmittel eine thermoelektrische Vorrichtung verwenden, die auf dem Peltier-Effekt basiert.

9. Verfahren nach einem der Ansprüche 1 bis 8, **dadurch gekennzeichnet, dass** die Kühlmittel bei einer Temperatur zwischen 3 °C und 4 °C liegen.

10. Verfahren nach einem der Ansprüche 1 bis 9, **dadurch gekennzeichnet, dass** die Temperatur in den Kühlmitteln über einen Zeitraum zwischen 5 Minuten und 60 Minuten aufrechterhalten wird.

11. Verfahren nach einem der Ansprüche 1 bis 10, **dadurch gekennzeichnet, dass** die Temperatur in den Erwärmungsmitteln in Schritt c) über einen Zeitraum zwischen 2 Minuten und 20 Minuten aufrechterhalten wird.

12. Verfahren nach Anspruch 1, **dadurch gekennzeichnet, dass** es aus den folgenden Schritten besteht:
a) Erwärmen eines lokalisierten Bereichs des menschlichen Körpers mit Erwärmungsmitteln, die bei einer Temperatur von nicht mehr als 44 °C liegen, für einen Zeitraum zwischen 2 und 10 Minuten,
b) anschließend Abkühlen dieses Bereichs mit Kühlmitteln, die bei einer Temperatur zwischen 3 °C und 5 °C liegen, für einen Zeitraum zwischen 5 und 60 Minuten, und
c) anschließend Erwärmen dieses Bereichs mit Erwärmungsmitteln, die bei einer Temperatur von nicht mehr als 44 °C liegen, für einen Zeitraum zwischen 2 und 20 Minuten.

13. Verfahren nach Anspruch 1, **dadurch gekennzeichnet, dass** es aus den folgenden Schritten besteht:
a) Erwärmen eines lokalisierten Bereichs des menschlichen Körpers mit Erwärmungsmitteln, die bei einer Temperatur von nicht mehr als 42 °C liegen, für einen Zeitraum zwischen 4 und 6 Minuten,
b) anschließend Abkühlen dieses Bereichs mit Kühlmitteln, die bei einer Temperatur zwischen 3 °C und 4 °C liegen, für einen Zeitraum zwischen 10 und 40 Minuten, und
c) anschließend Erwärmen dieses Bereichs mit Erwärmungsmitteln, die bei einer Temperatur von nicht mehr als 42 °C liegen, für einen Zeitraum zwischen 8 und 12 Minuten.

## Revendications

1. Procédé cosmétique pour la réduction du tissu adipeux sous-cutané dans une zone localisée d'un corps humain adulte, le procédé est constitué des étapes suivantes :
a) le chauffage d'une zone localisée du corps humain avec des moyens de chauffage qui sont à une température ne dépassant pas 45 °C,
b) ensuite, le refroidissement de cette zone avec des moyens de refroidissement qui sont à une température comprise entre 2 °C et 6 °C, **caractérisé par**,
c) ensuite, le chauffage de cette zone avec des moyens de chauffage qui sont à une température ne dépassant pas 45 °C.

2. Procédé selon la revendication 1, **caractérisé en ce que** la zone localisée d'un corps humain adulte est choisie dans le groupe constitué de l'abdomen, de la taille, du dos, des bras et des cuisses.

3. Procédé selon les revendications 1 ou 2, **caractérisé en ce que** les moyens de chauffage utilisent la radiofréquence, les micro-ondes, les ultrasons ou un dispositif thermoélectrique sur la base de l'effet Peltier.

4. Procédé selon la revendication 3, **caractérisé en ce que** les moyens de chauffage utilisent un dispositif thermoélectrique sur la base de l'effet Peltier.

5. Procédé selon l'une quelconque des revendications 1 à 4, **caractérisé en ce que** les moyens de chauffage sont à une température ne dépassant pas 42 °C.

6. Procédé selon l'une quelconque des revendications 1 à 5, **caractérisé en ce que** la température dans les moyens de chauffage à l'étape a) est maintenue pendant une durée comprise entre 2 minutes et 10 minutes.

7. Procédé selon l'une quelconque des revendications 1 à 6, **caractérisé en ce que** les moyens de refroidissement utilisent des solutions froides, l'air froid, ou un dispositif thermoélectrique sur la base de l'effet Peltier.

8. Procédé selon la revendication 7, **caractérisé en ce que** les moyens de refroidissement utilisent un dispositif thermoélectrique sur la base de l'effet Peltier.

9. Procédé selon l'une quelconque des revendications 1 à 8, **caractérisé en ce que** les moyens de refroidissement sont à une température comprise entre 3 °C et 4 °C.

10. Procédé selon l'une quelconque des revendications 1 à 9, **caractérisé en ce que** la température dans les moyens de refroidissement est maintenue pendant une durée comprise entre 5 minutes et 60 minutes.

11. Procédé selon l'une quelconque des revendications 1 à 10, **caractérisé en ce que** la température dans les moyens de chauffage à l'étape c) est maintenue pendant une durée comprise entre 2 minutes et 20 minutes.

12. Procédé selon la revendication 1, caractérisé **en ce qu**'il est constitué des étapes suivantes :
a) le chauffage d'une zone localisée du corps humain avec des moyens de chauffage qui sont à une température ne dépassant pas 44 °C, pour une durée comprise entre 2 et 10 minutes,
b) ensuite, le refroidissement de cette zone avec des moyens de refroidissement qui sont à une température comprise entre 3 °C et 5 °C pendant une durée comprise entre 5 et 60 minutes, et
c) ensuite, le chauffage de cette zone avec des moyens de chauffage qui sont à une température ne dépassant pas 44 °C pendant une durée comprise entre 2 et 20 minutes.

13. Procédé selon la revendication 1, caractérisé **en ce qu**'il est constitué des étapes suivantes :
a) le chauffage d'une zone localisée du corps humain avec des moyens de chauffage qui sont à une température ne dépassant pas 42 °C, pour une durée comprise entre 4 et 6 minutes,
b) ensuite, le refroidissement de cette zone avec des moyens de refroidissement qui sont à une température comprise entre 3 °C et 4 °C pendant une durée comprise entre 10 et 40 minutes, et
c) ensuite, le chauffage de cette zone avec des moyens de chauffage qui sont à une température ne dépassant pas 42 °C pendant une durée comprise entre 8 et 12 minutes.
